(19) Europäisches Patentamt

European Patent Office

Office européen des brevets

(11) **EP 0 484 448 B2**

(12) **NOUVEAU FASCICULE DE BREVET EUROPEEN**

(45) Date de publication et mention de la décision concernant l'opposition:
**02.01.1997 Bulletin 1997/01**

(45) Mention de la délivrance du brevet:
**15.12.1993 Bulletin 1993/50**

(21) Numéro de dépôt: **90912572.6**

(22) Date de dépôt: **03.08.1990**

(51) Int. Cl.$^6$: **B01J 2/04**, C04B 18/02, C04B 38/00

(86) Numéro de dépôt international:
**PCT/FR90/00588**

(87) Numéro de publication internationale:
**WO 91/01798 (21.02.1991 Gazette 1991/05)**

(54) **PROCEDE DE FABRICATION DE POUDRES ORDONNEES PAR PULVERISATION A PARTIR D'AU MOINS DEUX POPULATIONS DE PARTICULES**

HERSTELLUNGSVERFAHREN FÜR EIN PULVER AUS GEORDNETEN TEILCHEN DURCH ZERSTÄUBUNG AUSGEHEND VON MINDESTENS ZWEI VERSCHIEDENEN KORNGRÖSSEN

PROCESS FOR PRODUCING ORGANISED POWDERS BY SPRAYING FROM AT LEAST TWO SETS OF PARTICLES

(84) Etats contractants désignés:
**AT BE CH DE DK ES FR GB IT LI LU NL SE**

(30) Priorité: **04.08.1989 FR 8910565**

(43) Date de publication de la demande:
**13.05.1992 Bulletin 1992/20**

(73) Titulaire: **LVMH RECHERCHE
92752 Nanterre (FR)**

(72) Inventeurs:
• **MEYBECK, Alain
Les Poissons
Appartement 242
F-92400 Courbevoie (FR)**
• **ANTOINE, Philippe
F-53200 Château-Gontier (FR)**

(74) Mandataire: **Portal, Gérard et al
Cabinet Beau de Loménie
158, rue de l'Université
75340 Paris Cédex 07 (FR)**

(56) Documents cités:
| | |
|---|---|
| EP-A- 0 149 098 | EP-A- 0 154 189 |
| EP-A- 0 224 659 | EP-A- 0 347 769 |
| EP-A- 0 510 805 | EP-B- 0 149 098 |
| EP-B- 0 299 344 | BE-A- 644 651 |
| DE-A- 2 148 224 | DE-A- 2 238 903 |
| DE-B- 2 214 410 | DE-B- 2 614 261 |
| GB-A- 1 412 790 | US-A- 3 325 425 |
| US-A- 3 907 734 | US-A- 3 985 703 |

• **Powder Technology 11 (1975), pages 42-44**
• **Powder Technology 25 (1980), pages 115-119**

EP 0 484 448 B2

**Description**

La présente invention concerne essentiellement un procédé de fabrication de poudres ordonnées à partir de particules de tailles différentes appartenant à au moins deux populations de particules de tailles sensiblement homogènes.

Dans le présent document, on entend par l'expression "poudre ordonnée" l'assemblage régulier de particules de tailles différentes, des particules de petites tailles, ou "particules filles", venant se fixer en ordre à la surface d'une particule de plus grande taille, ou "particule mère" ou "noyau", de manière à recouvrir celle-ci en une ou plusieurs couches de façon partielle ou totale.

Dans l'état de la technique antérieure, on connait divers procédés pour la fabrication de poudres ordonnées obtenues à partir de particules de tailles différentes. Par exemple le document JP-A-62-083 029 NARA décrit un procédé et un dispositif permettant de préparer des poudres ordonnées à partir de deux populations de particules de tailles homogènes par une technique dite à choc par percussion. Les particules noyaux ont en général un diametre moyen d'environ 0,1 μm à 100 μm et les particules filles ont en général un diamétre moyen d'environ 0,01 μm à 10 μm. Par ce procédé, on obtient une bonne adhésion des particules filles aux particules noyaux, selon un recouvrement sensiblement uniforme, l'ensemble constituant un système généralement désigné par l'expression "poudre hybride".

Par ailleurs, divers articles ont été publiés dans la littérature sur les mélanges ordonnés. On peut se reporter en particulier à la revue Powder Technology 11 (1975) 41-44 ou 25 (1980) 115-119.

Cependant, les techniques utilisées antérieurement sont peu satisfaisantes. En particulier, elles sont complexes et coûteuses, et donc difficilement utilisables à l'echelle industrielle.

La présente invention a donc pour but de résoudre le nouveau problème technique consistant en la fourniture d'un procédé de fabrication de poudres ordonnées d'une conception simple, permettant de réaliser des poudres ordonnées de manière reproductible, dont les paramètres du procédé peuvent être adaptés à la demande industrielle et à faible coût.

Ce nouveau problème technique est resolu pour la première fois d'une manière extrêmement simple par la présente invention, ce qui le rend utilisable à l'échelle industrielle.

Ainsi, la présente invention fournit un procédé de fabrication de poudres ordonnées à partir de particules de tailles différentes appartenant à au moins deux populations de particules de tailles sensiblement homogènes, comprenant respectivement au moins une population de particules noyaux et au moins une population de particules filles de taille plus réduite que celle des particules noyaux, caractérisé en ce qu'on réalise dans une première étape une dispersion sensiblement homogène de chaque population dans un liquide de dispersion ; et, dans une deuxième étape, on pulvérise ladite dispersion dans des conditions appropriées permettant d'aboutir à la formation de ladite poudre ordonnée.

Dans le cadre de la présente description et des revendications, le terme "pulvérisation" doit être compris au sens large, ce terme couvrant notamment les termes "nébulisation" et "atomisation".

Selon un mode de réalisation particulier, la pulvérisation de ladite dispersion est effectuée dans une enceinte dans des conditions de pression et de température qui permettent L'évaporation du liquide de dispersion, aboutissant ainsi à la formation de ladite poudre ordonnée.

Avantageusement, la pulvérisation est effectuée dans un fluide gazeux porté à une température suffisante pour réaliser une évaporation du liquide de dispersion.

La température du fluide gazeux est de préférence supérieure à la température d'ébullition du liquide de dispersion.

En particulier, le fluide gazeux est constitué par de l'air.

On remarquera que, bien entendu pour la mise en oeuvre de la présente invention, le liquide de dispersion ne doit pas être susceptible de dissoudre lesdites particules.

Selon une variante de réalisation particulière du procédé selon l'invention, la dispersion précitée est réalisée dans un liquide unique.

Avantageusement, le liquide de dispersion est constitué par de l'eau ou une solution aqueuse.

Selon une autre variante de réalisation particulière, la dispersion de chaque population est réalisée dans un liquide de dispersion particulier et les deux dispersions sont ensuite mélangées de manière homogène soit préalablement à la pulvérisation, soit au moment de la pulvérisation.

Selon une autre variante de réalisation, on ajoute au liquide de dispersion, préalablement à l'introduction des particules, une substance mouillante, telle qu'un tensioactif, comme par exemple le produit commercialisé sous l'appellation TWEEN 20 (ICI Europe U.K.) à la concentration de 1% en poids de matière sèche, c'est-à-dire de la quantité totale de particules à disperser.

D'une façon générale, la teneur en matière sèche dans le liquide de dispersion, c'est-à-dire le rapport de la quantité en poids de particules noyaux et de particules filles à la quantité en poids de liquide de dispersion, pourra être comprise entre 5 et 40 %, de préférence entre 10 et 20 %, et encore de préférence entre 10 et 15 %.

Selon une caractéristique particulière, le diamètre moyen des particules filles est avantageusement inférieur ou égal à environ 1/5è du diamètre moyen des particules noyaux.

Selon une autre caractéristique, la proportion relative en poids des particules filles relativement aux particules noyaux peut varier dans de larges limites, et dépend de la structure recherchée de la poudre ordonnée.

Plus précisément, la proportion relative en poids (R) des particules filles relativement aux particules noyaux sera déterminée en fonction du nombre de particules filles que l'on cherchera à faire adhérer sur une particule noyau et des masses moyennes des particules filles et des particules noyaux, selon la formule :

$$R = \frac{nM}{M_o}$$

dans laquelle n représente le nombre moyen de particules filles adhérant sur une particule noyau, M représente la masse moyenne d'une particule fille et $M_o$ représente la masse moyenne d'une particule noyau.

Le nombre de particules filles adhérant sur une particule noyau dépend essentiellement de la surface occupée par chaque particule fille sur une particule noyau, de la surface occupée sur la particule noyau (le recouvrement de la particule noyau peut être partiel ou total) et du nombre de couches de particules filles sur une particule noyau (structure monocouche ou multicouche).

Dans le cas d'une structure monocouche, et pour un recouvrement total des particules noyaux, le nombre théorique de particules filles susceptibles d'adhérer sur une particule noyau est égal au rapport de la surface totale d'une particule noyau A la surface occupée par une particule fille.

Ce rapport permet donc de calculer la proportion pondérale théorique de chacune des deux populations de particules, en pourcentage par rapport au poids total de poudre.

Pour la mise en oeuvre de la présente invention, on choisira donc des proportions pondérales proches des valeurs théoriques. Toutefois, pour tenir compte en particulier d'une certaine variabilité de forme des particules, on pourra s'écarter plus ou moins de cette valeur théorique pour obtenir un résultat souhaité.

A titre d'exemples, on a résumé au tableau I le calcul théorique de la proportion relative en poids (ou en masse) des particules filles relativement aux particules noyaux dans les deux cas les plus courants, où les particules filles sont sensiblement de forme sphérique et les particules noyaux sont sensiblement de forme sphérique ou de forme cylindrique telle qu'un disque.

Dans les deux cas, on a considéré que la surface de particule noyau occupée par une particule fille est sensiblement égale à la surface projetée de ladite particule fille, c'est-à-dire en fait sa surface équatoriale.

Dans le cas où les particules noyaux ont une forme de disque, on a considéré dans le calcul que la hauteur h du disque était très faible, et qu'elle était donc négligeable par rapport au diamètre du disque.

Le principe général de ce calcul peut être utilisé quelles que soient les formes géométriques des particules filles et des particules noyaux.

En outre, dans les calculs que l'on fait, on tiendra compte de La disparité de la taille des particules en retenant notamment des tailles moyennes et des masses moyennes pour les particules.

Ainsi, selon un mode de réalisation préféré, le pourcentage en poids des particules filles exprimé par rapport à la matière sèche totale est de préférence voisin ce ou égal au pourcentage théorique donné par la formule :

$$C = \frac{S_o\rho V}{S_o\rho V + \rho_o V_o S} \times 100$$

dans Laquelle S, $S_o$, $\rho$, $\rho_o$, V et $V_o$ ont les significations indiquées au Tableau I ci-après.

TABLEAU I

| | $S_0$ | $S$ | $n = \dfrac{S_0}{S}$ | $M_0 = \rho_0 V_0$ | $M = \rho V$ | $R = \dfrac{nM}{M_0}$ |
|---|---|---|---|---|---|---|
| fille/noyau | $S_0$ | $S$ | | | | |
| sphère/sphère | $\pi D^2$ | $\dfrac{\pi d^2}{4}$ | $4\dfrac{D^2}{d^2}$ | $\rho_0 \pi \dfrac{D^3}{6}$ | $\rho \pi \dfrac{d^3}{6}$ | $4\dfrac{\rho d'}{\rho_0 D}$ |
| sphère/disque | $\pi \dfrac{D^2}{2}$ | $\dfrac{\pi d^2}{4}$ | $2\dfrac{D^2}{d^2}$ | $\rho_0 \pi \dfrac{D^2 h}{4}$ | $\rho \pi \dfrac{d^3}{6}$ | $\dfrac{4}{3}\dfrac{\rho}{\rho_0}\dfrac{d}{h}$ |

$S_0$ = surface totale d'une particule noyau

$S$ = portion de surface de particule noyau occupée par une particule fille

$n$ = nombre de particules filles pour une particule noyau

$M_0$ = masse d'une particule noyau

$M$ = masse d'une particule fille

$R$ = portion relative en poids (ou en masse) de particules filles relativement aux particules noyaux

$D$ = diamètre d'une particule noyau

$d$ = diamètre d'une particule fille

$\rho_0$ = masse volumique des particules noyaux

$\rho$ = masse volumique des particules filles

$h$ = hauteur d'une particule noyau (cas d'un disque)

$V$ = volume d'une particule fille

$V_0$ = volume d'une particule noyau

Selon un mode de réalisation particulier, le liquide de dispersion précité contient un composé liant permettant de favoriser l'accrochage des particules filles sur les particules noyaux.

Avantageusement, ce composé liant est dissous dans le liquide de dispersion servant de vecteur.

Selon une autre variante, le composé liant peut être simplement mis en suspension dans le liquide de dispersion servant de vecteur.

A titre d'exemples, on peut utiliser comme composé liant la carboxyméthylcellulose, l'hydroxypropylmethylcellulose, la méthylcellulose, la polyvinylpyrrolidone, l'éthylcellulose, l'hybroxypropylcellulose, ou des polyméthacrylates.

Le composé liant peut être lui-même additionné d'agents plastifiants ou d'agents anticollage pour éviter l'agrégation des particules de poudre ordonnée.

Selon une autre variante de réalisation du procédé selon l'invention, on obtient l'adhésion entre les particules filles et les particules noyaux en utilisant un matériau pour réaliser au moins un des types de population de particules qui peut être ramolli ou fondu à la température du fluide gazeux utilisé pour évaporer le liquide de dispersion.

4

En outre, dans ce cas, la température du fluide gazeux, supérieure à la température de ramollissement des particules filles et/ou des particules noyaux, est généralement suffisamment élevée pour que l'évaporation du liquide de dispersion soit rapide et totale et que les particules parvenant au cyclone de l'installation soient parfaitement sèches.

Le procédé conforme à l'invention peut par exemple être mis en oeuvre dans une installation d'évaporation classique comportant une enceinte de pulvérisation reliée d'une part à une cuve de dispersion des particules noyaux et des particules filles et d'autre part à un cyclone, telle qu'une installation d'atomisation. La dispersion est introduite sous pression (gaz comprimé à environ 3 à 10 bars, de préférence 7 bars) dans l'enceinte de pulvérisation par exemple à travers l'orifice d'une buse, les gouttelettes ainsi obtenues étant séchées par un gaz chaud (par exemple de l'air à environ 150-250°C).

Les applications de telles poudres ordonnées sont bien connues à l'homme de l'art et décrites dans la littérature. En particulier, ces poudres ordonnées peuvent être utilisées dans le domaine cosmétique, pharmaceutique, phytosanitaire, agroalimentaire, dans le domaine des pesticides, des peintures ou de la métallurgie.

A titre d'exemples, les particules noyaux peuvent être constituées par ou contenir les produits suivants : polyester, polyéthylène polystyrène, polyméthylméthacrytate, cellulose, Nylon 6, Nylon 12, Téflon, polymère de chlorure de vinyle ou encore une résine époxy, acrylique, méthacrylique.

De même, les particules filles peuvent être constituées par ou contenir les produits suivants : une poudre minérale comme par exemple talc, kaolin, mica, vermiculite silice ; une poudre organique comme par exemple une poudre de Nylon, de polyéthyléne ; un pigment minéral comme par exemple oxyde de titane, oxyde de zinc, oxyde de fer, titanate de fer, noir de carbone, violet de manganèse, oxyde de chrome, bleu outremer, bleu de Prusse.

Avantageusement, les particules noyaux et les particules filles sont choisies parmi les couples suivants de populations de particules : nylon-oxyde de titane, nylon-silice, nylon-oxyde de fer jaune, nylon-bleu outremer, polystyrène-oxyde de titane, polyéthylène-oxyde de titane, polyéthylène-oxyde de fer jaune, polyéthylène-oxyde de fer noir, polyéthylène-bleu outremer.

Outre l'avantage économique, déjà mentionné, par rapport aux procédés connus, le procédé selon l'invention présente de nombreux autres avantages. Notamment, il permet d'obtenir un recouvrement très régulier des particules noyaux quelle que soit leur forme, y compris les formes en plaquettes. Il permet également de réaliser très aisément des poudres ordonnées constituées de particules filles et noyaux de dureté comparable, au besoin au moyen d'un liant.

D'autres buts, caractéristiques et avantages de l'invention apparaîtront également à la lumière de la description explicative qui va suivre faite en référence aux exemples de réalisation de l'invention donnés simplement à titre d'illustration et qui ne sauraient donc en aucune façon limiter la portée de l'invention.

Exemple 1

On utilise comme particules noyaux 93 g de poudre (particules sensiblement sphériques ou billes) de Nylon 12 ayant un diamètre moyen égal à environ 5 μm et une masse volumique d'environ 1,02 g/cm$^3$ et comme particules filles 7 g de poudre (particules sensiblement sphériques) de bioxyde de titane ayant un diamètre moyen de particules d'environ 0,02 μm et une masse volumique de 4 g/cm$^3$ environ.

Ces deux groupes de particules noyaux et de particules filles sont mis en suspension de manière homogène dans une solution aqueuse contenant 899 g d'eau et 1 g de TWEEN 20 (ICI Europe U.K).

On pulvérise ensuite à l'intérieur d'un atomiseur ladite dispersion dans un fluide gazeux constitué par de l'air porté à une température d'environ 230°C, à la pression de 7 bars pour un débit de liquide de 5 litres par heure.

On obtient ainsi une poudre ordonnée dont les particules noyaux comportent à leur surface une pluralité de particules filles. Grâce à la proportion relative utilisée dans le cas présent, les particules filles constituent un revêtement continu sur la surface des particules noyaux.

En utilisant le protocole expérimental décrit à l'exemple 1, on a réalisé un certain nombre de poudres ordonnées en utilisant différents types de matériaux pour les particules noyaux et filles, et en faisant varier notamment les proportions relatives en poids de particules noyaux relativement aux particules filles.

Les résultats de ces essais ont été reportés au tableau II et montrent que l'on obtient selon des conditions expérimentales choisies un revêtement de qualité moyenne à excellente.

Dans ce dernier cas, on obtient un recouvrement total et régulier.

TABLEAU II

| | Composition | | | Liquide de dispersion (solvant) | P pourcentage matière sèche | Gaz vecteur | Température °C | Résultat qualité du revêtement |
|---|---|---|---|---|---|---|---|---|
| | Nature | Forme/$\phi$ | Quantité en poids (1) | | | | | |
| Particule noyau | Nylon 12 SP 500 | billes 5 µ | 90 % | $H_2O$ | 10 % | air | 200-230 | ★★★ |
| Particule fille | TiO$_2$ (P25) | billes 0,02 µ | 10 % | | | | | |
| Particule noyau | Nylon 12 SP 500 | billes 5 µ | 95 % | $H_2O$ | 10 % | air | 150-200 | ★★★ |
| Particule fille | SiO$_2$ (Aérosil 200) | billes 0,012 µ | 5 % | | | | | |
| Particule noyau | Polystyrène | billes 6 µ | 85 % | $H_2O$ | 10 % | air | 150-200 | ★★ |
| Particule fille | TiO$_2$ | billes 0,02 µ | 15 % | | | | | |
| Particule noyau | Nylon 12 flakes | disques 10 µ | 90 % | $H_2O$ | 10 % | air | 150-200 | ★★ |
| Particule fille | SiO$_2$ (Aérosil 200) | billes 0,012 µ | 10 % | | | | | |
| Particule noyau | Nylon 12 flakes | disques 12 µ | 70 % | $H_2O$ | 10 % | air | 180-230 | ★★ |
| Particule fille | TiO$_2$ | billes 0,02 µ | 30 % | | | | | |
| Particule noyau | Polyéthylène | billes 10 µ | 70 % | $H_2O$ | 10 % | air | 120-150 | ★ |
| Particule fille | TiO$_2$ | billes 0,02 µ | 30 % | | | | | |
| Particule noyau | Nylon 12 flakes | disques 12 µ | 70 % | $H_2O$ | 10 % | air | 150-180 | ★★ |
| Particule fille | oxyde de fer jaune | aiguilles 0,5 µ | 30 % | | | | | |

NYLON 12 SP500 : TORAY (Japon)

TiO$_2$ (P25) : DEGUSSA (France)

AEROSIL 200 : DEGUSSA (France)

NYLON 12 Flakes : SEITETSU (Japon)

EP 0 484 448 B2

TABLEAU II (suite)

| | Composition | | | Liquide de dispersion (solvant) | P pourcentage matière sèche | Gaz vecteur | Température °C | Résultat qualité du revêtement |
|---|---|---|---|---|---|---|---|---|
| | Nature | Forme/φ | Quantité en poids (1) | | | | | |
| Particule noyau | Nylon 12 (SP 500) | billes 5 μ | 70 % | $H_2O$ | 10 % | air | 150-190 | ** |
| Particule fille | oxyde de fer jaune | aiguilles 0,5 μ | 30 % | | | | | |
| Particule noyau | polyéthylène | billes 10 μ | 70 % | $H_2O$ | 10 % | air | 130-150 | * |
| Particule fille | oxyde de fer noir | billes 0,2 μ | 30 % | | | | | |

\* moyen
\*\* bon
\*\*\* excellent

$$R = \frac{\text{Quantité en poids de particules filles}}{\text{Quantité en poids de particules mères}}$$

$$P = \frac{\text{Quantité en poids de particules filles + mères}}{\text{Quantité en poids de liquide de dispersion}}$$

(1) Les quantités indiquées sont les proportions pondérales, par rapport à la matière sèche.

## Exemple 2

On opère selon le protocole expérimental de L'exemple 1, si ce n'est qu'on introduit dans la solution aqueuse de dispersion une certaine quantité d'un liant.

Les résultats obtenus sont présentés au tableau III sous une forme semblable à celle du tableau II.

Ce mode de réalisation permet notamment de pallier aux inconvénients des procédés connus, dans le cas où les particules filles se fixent difficilement sur les particules noyaux.

Naturellement, l'invention comprend tous les moyens constituant des équivalents techniques des moyens décrits ainsi que leurs diverses combinaisons.

TABLEAU III

| | Composition | | | Q (2) | Liquide de dispersion (solvant) | P pourcentage matière sèche | Gaz vecteur | Température °C | Résultat qualité du revêtement |
|---|---|---|---|---|---|---|---|---|---|
| | Nature | Forme/φ | Quantité en poids | | | | | | |
| Particule noyau | polyéthylène | billes 8 µ | 60 % | | $H_2O$ | 10 % | air | 120-150 | ** |
| Particule fille | bleu outremer | 0,3-0,8 µ | 40 % | | | | | | |
| Liant | H.P.M.C | | | 0,05 % | | | | | |
| Particule noyau | Nylon 12 | disques 10 µ | 70 % | | $H_2O$ | 10 % | air | 150-190 | ** |
| Particule fille | bleu outremer | 0,3-0,8 µ | 30 % | | | | | | |
| Liant | C.M.C | | | 0,05 % | | | | | |
| Particule noyau | Nylon 12 (SP 500) | billes 5 µ | 70 % | | $H_2O$ | 10 % | air | 150-200 | * |
| Particule fille | bleu outremer | 0,3-0,8 µ | 30 % | | | | | | |
| Liant | H.P.M.C | | | 0,05 % | | | | | |
| Particule noyau | polyéthylène | disques 20 µ | 75 % | | $H_2O$ | 10 % | air | 120-150 | ** |
| Particule fille | bleu outremer | 0,3-0,8 µ | 25 % | | | | | | |
| Liant | H.P.M.C | | | 0,05 % | | | | | |

* moyen
** bon
*** excellent

Q = Pourcentage de liant dans la solution aqueuse
C.M.C = Carboxyméthylcellulose
H.P.M.C = Hydroxypropylméthylcellulose

**Revendications**

1. Procédé de fabrication de poudres ordonnées obtenues à partir de particules de tailles différentes appartenant à au moins deux populations de particules de tailles sensiblement homogènes comprenant respectivement au moins une population de particules noyaux et au moins une population de particules filles, caractérisé en ce qu'on réalise dans une première étape une dispersion sensiblement homogène de chaque population dans un liquide de dispersion ; et, dans une deuxième étape, on pulvérise ladite dispersion dans des conditions appropriées permettant d'aboutir à la vaporisation du liquide de dispersion et à la formation de ladite poudre ordonnée.

2. Procédé selon la revendication 1, caractérisé en ce que la pulvérisation de la dispersion précitée est effectuée dans une enceinte dans des conditions de pression et de température qui permettent d'aboutir à ladite vaporisation du liquide de dispersion et à la formation de ladite poudre ordonnée.

3. Procédé selon la revendication 1 ou 2, caractérisé en ce que la pulvérisation de la dispersion précitée est effectuée dans un fluide gazeux porté à une température suffisante pour évaporer le liquide de dispersion.

4. Procédé selon l'une des revendications 1 à 3, caractérisé en ce que la dispersion est réalisée dans un liquide de dispersion unique.

5. Procédé selon l'une des revendications 1 à 4, caractérisé en ce que le liquide de dispersion est constitué par de l'eau ou une solution aqueuse.

6. Procédé selon l'une des revendications 1 à 5, caractérisé en ce que le fluide gazeux est constitué par de l'air.

7. Procédé selon l'une des revendications 1 à 6, caractérisé en ce que le diamètre moyen des particules filles est inférieur ou égal à environ 1/5ème du diamètre moyen des particules noyaux.

8. Procédé selon l'une des revendications précédentes, caractérisé en ce qu'il comprend préalablement à l'introduction des particules l'adjonction, au liquide de dispersion, d'une quantité efficace d'une substance mouillante, telle qu'un tensioactif.

9. Procédé selon l'une des revendications précédentes, caractérisé en ce que le pourcentage en poids des particules filles exprimé par rapport à la matière sèche totale est voisin de ou égal au pourcentage théorique donné par la formule:

$$C = \frac{S_o \rho V}{S_o \rho V + \rho_o V_o S} \times 100$$

dans laquelle $S$ représente la portion de surface de particule noyau occupée par une particule fille, $S_o$ représente la surface totale d'une particule noyau, $\rho$ représente la masse volumérique des particules filles, $\rho_o$ représente la masse volumique des particules noyaux, $V$ représente le volume d'une particule fille et $V_o$ représente le volume d'une particule noyau.

10. Procédé selon l'une des revendications précédentes, caractérisé en ce que le liquide de dispersion précité contient au moins un composé liant capable de favoriser la liaison entre les particules filles et les particules noyaux.

11. Procédé selon la revendication 10, caractérisé en ce que le composé liant est dissous dans le liquide de dispersion servant de vecteur.

12. Procédé selon la revendication 10 ou 11, caractérisé en ce que le composé liant est constitué par de la carboxyméthylcellulose ou de l'hydroxypropylméthylcellulose.

13. Procédé selon l'une des revendications précédentes, caractérisé en ce que les particules filles et/ou les particules noyaux sont constituées en un matériau qui est ramolli ou fondu à la température du fluide gazeux utilisé pour évaporer le liquide de dispersion.

14. Procédé selon l'une des revendications précédentes, caractérisé en ce qu'au moins une population de particules noyaux est constituée par ou contient un polymère organique, en particulier choisi parmi un polyester, le polyéthylène, le polystyrène, le polyméthylméthacrylate, la cellulose, le nylon 6, le nylon 12, le Téflon, un polymère de chlorure de vinyle, une résine époxy, une résine acrylique, une résine méthacrylique ; les particules filles précitées formant une monocouche sensiblement uniforme enveloppant la surface de chacune des particules noyaux, la dispersion précitée ayant lieu dans un liquide de dispersion dans un rapport des particules filles aux particules noyaux approprié pour former ladite monocouche de particules filles sur les particules noyaux.

**15.** Procédé selon la revendication 14, caractérisé en ce que les particules filles sont constituées par ou contiennent une poudre minérale, en particulier talc, kaolin, mica, vermiculite, silice.

**16.** Procédé selon l'une des revendications précédentes, caractérisé en ce que les particules filles comprennent un pigment, en particulier oxyde de titane, oxyde de zinc, oxyde de fer, titanate de fer, noir de carbone, violet de manganèse, oxyde de chrome bleu outremer, bleu de prusse.

**17.** Procédé selon l'une des revendications précédentes, caractérisé en ce que les particules filles sont constituées par ou contiennent une poudre organique, en particulier une poudre de nylon, de polyéthyléne.

**18.** Procédé selon l'une des revendications précédentes, caractérisé en ce que les particules noyaux et les particules filles sont choisies par les couples suivants de populations de particules : Nylon-oxyde de titane, nylon-silice, nylon-oxyde de fer jaune, nylon-bleu outremer, polystyrène-oxyde de titane, polyéthylène-oxyde de titane, polyéthylene-oxyde de fer jaune, polyéthylène-oxyde de fer noir, polyéthylène-bleu outremer.

**Claims**

**1.** Process for producing organized powders obtained from particles of different sizes belonging to at least two sets of particles of substantially homogeneous sizes comprising, respectively, at least one set of core particles and at least one set of satellite particles, characterized in that, in a first step, a substantially homogeneous dispersion of each set is made in a dispersing liquid; and, in a second step, said dispersion is sprayed in suitable conditions to obtain the evaporation of the dispersing liquid and the formation of said organized powder.

**2.** Process according to claim 1, characterized in that the spraying of the above cited dispersion is performed inside an enclosure in conditions of pressure and temperature such as to obtain said evaporation of the dispersing liquid and the formation of said organized powder.

**3.** Process according to claim 1 or 2, characterized in that the spraying of the above cited dispersion is performed in a gaseous fluid brought to a sufficient temperature for evaporating the dispersing liquid.

**4.** Process according to one of claims 1 to 3, characterized in that the dispersion is produced in one sole dispersing liquid.

**5.** Process according to one of claims 1 to 4, characterized in that the dispersing liquid is constituted by water or an aqueous solution.

**6.** Process according to one of claims 1 to 5, characterized in that the gaseous fluid is constituted by air.

**7.** Process according to one of claims 1 to 6, characterized in that the mean diameter of the satellite particles is less than or equal to about one fifth of the mean diameter of the core particles.

**8.** Process according to any one of the preceding claims, characterized in that it comprises, prior to the introduction of the particles, the addition to the dispersing liquid of an efficient amount of a wetting substance, such as a surfactant.

**9.** Process according to one of the preceding claims, characterized in that the percentage by weight of the satellite particles expressed with respect to the total dry material is preferably dose or equal to the theoretical percentage given by the formula:

$$C = \frac{S_o \rho V}{S_o \rho V + \rho_o V_o S} \times 100$$

in which $S$ is the portion of surface of core particle occupied by a satellite particle, $S_o$ is the total surface of a core particle, $\rho$ is the density of the satellite particles, $\rho_o$ is the density of the core particles, $V$ is the volume of a satellite

particle and $V_o$ is the volume of a core particle.

10. Process according to one of the preceding claims, characterized in that the above cited dispersing liquid contains at least one binding compound designed to help the satellite particles to adhere to the core particles.

11. Process according to claim 10, characterized in that the binding compound is dissolved in the dispersing liquid serving as a vector.

12. Process according to claim 10 or 11, characterized in that the binding compound is constituted by carboxymethylcellulose or by hydroxypropylmethylcellulose.

13. Process according to one of the preceding claims, characterized in that the satellite particles and/or the core particles are constituted in a material which is softened or melted at the temperature of the gaseous fluid used for evaporating the dispersing liquid.

14. Process according to one of the preceding claims, characterized in that at least one set of core particles is constituted by or contains an organic polymer, in particular selected from a polyester, polyethylene, polystyrene, polymethylmethacrylate, cellulose, Nylon 6, Nylon 12, Teflon, a vinyl chloride polymer, an epoxy resin, an acrylic resin, a methacrylic resin; the above cited satellite particles forming a monolayer substantially uniform, coating the surface of each of the core particles, the above cited dispersion taking place in a dispersing liquid in an appropriate ratio between the satellite particles and the core particles to form said satellite particle monolayer on the core particles.

15. Process according to claim 14, characterized in that the satellite particles are constituted by or contain a mineral powder, in particular talc, kaolin, mica, vermiculite, silica.

16. Process according to one of the preceding claims, characterized in that the satellite particles contain a pigment, in particular titanium oxide, zinc oxide, iron oxide, iron titanate, carbon black, manganese purple, chromium oxide, ultramarine blue, Prussian blue.

17. Process according to one of the preceding claims, characterized in that the satellite particles are constituted by or contain an organic powder, in particular a Nylon or polyethylene powder.

18. Process according to one of the preceding claims, characterized in that the core particles and the satellite particles are selected from the following couples of sets of particles : Nylon-titanium oxide, Nylon-silica, Nylon-yellow iron oxide, Nylon-ultramarine blue, polystyrene-titanium oxide, polyethylene-titanium oxide, polyethylene-yellow iron oxide, polyethylene-black iron oxide, polyethylene-ultramarine blue.

**Patentansprüche**

1. Verfahren zur Herstellung von geordneten Pulvern, welche aus Teilchen unterschiedlicher Größen, die mindestens zwei Teilchenmassen von im wesentlichen homogenen Größen zugehören, umfassend mindestens eine Masse aus Kernteilchen und mindestens eine Masse aus Tochterteilchen, erhalten werden, dadurch gekennzeichnet, daß man in einer ersten Stufe eine im wesentlichen homogene Dispersion jeder Masse in einer Dispergierflüssigkeit herstellt und man in einer zweiten Stufe diese Dispersion unter entsprechenden Bedingungen pulverisiert, sodaß man die Eindampfung der Dispergierflüssigkeit und die Bildung des geordneten Pulvers bewirken kann.

2. Verfahren nach Anspruch 1, dadurch gekennzeichnet, daß die Pulverisierung der genannten Dispersion in einem Gehäuse unter Druck- und Temperaturbedingungen ausgeführt wird, die die Erzielung der Eindampfung der Dispergierflüssigkeit und der Bildung des geordneten Pulvers ermöglichen.

3. Verfahren nach Anspruch 1 oder 2, dadurch gekennzeichnet, daß die Pulverisierung der genannten Dispersion in einem gasförmigen Fluid ausgeführt wird, das auf eine zur Eindampfung der Dispergierflüssigkeit ausreichende Temperatur gebracht wird.

4. Verfahren nach einem der Ansprüche 1 bis 3, dadurch gekennzeichnet, daß die Dispersion in einer einzigen Dispergierflüssigkeit hergestellt wird.

5. Verfahren nach einem der Ansprüche 1 bis 4, dadurch gekennzeichnet, daß die Dispergierflüssigkeit aus Wasser oder einer wässerigen Lösung besteht.

6. Verfahren nach einem der Ansprüche 1 bis 5, dadurch gekennzeichnet, daß das gasförmige Fluid aus Luft besteht.

7. Verfahren nach einem der Ansprüche 1 bis 6, dadurch gekennzeichnet, daß der mittlere Durchmesser der Tochterteilchen kleiner oder gleich etwa 1/5 des mittleren Durchmessser der Kornteilchen ist.

8. Verfahren nach einem der vorhergehenden Ansprüche, dadurch gekennzeichnet, daß es vor der Einbringung der Teilchen die Zugabe einer wirksamen Menge eines Netzmittels, wie eines Tensids, zur Dispergierflüssigkeit umfaßt.

9. Verfahren nach einem der vorhergehenden Ansprüche, dadurch gekennzeichnet, daß der Gewichtsprozentsatz der Tochterteilchen, ausgedrückt im Verhältnis zum Gesamttrockengewicht, nahe oder gleich dem theoretischen Prozentsatz liegt, der durch die Formel

$$C = \frac{S_o \rho V}{S_o \rho V + \rho_o V_o S} \times 100$$

gegeben ist, worin S den von einem Tochterteilchen eingenommenen Oberflächenanteil des Kernteilchens darstellt, $S_0$ die Gesamtfläche eines Kernteilchens darstellt, $\rho$ die volumsbezogene Masse der Tochterteilchen darstellt, $\rho_0$ die volumsbezogene Masse der Kernteilchen darstellt, V das Volumen eines Tochterteilchens darstellt und $V_0$ das Volumen eines Kernteilchens darstellt.

10. Verfahren nach einem der vorhergehenden Ansprüche, dadurch gekennzeichnet, daß die Dispergierflüssigkeit mindestens eine Bindemittelverbindung enthält, die die Bindung zwischen den Tochterteilchen und den Kernteilchen begünstigen kann.

11. Verfahren nach Anspruch 10, dadurch gekennzeichnet, daß die Bindemittelverbindung in der als Träger dienenden Dispergierflüssigkeit gelöst wird.

12. Verfahren nach Anspruch 10 oder 11, dadurch gekennzeichnet, daß die Bindemittelverbindung aus Carboxymethylcellulose oder Hydroxypropylcellulose besteht.

13. Verfahren nach einem der vorhergehenden Ansprüche, dadurch gekennzeichnet, daß die Tochterteilchen und/oder die Kernteilchen aus einer Substanz bestehen, die bei der Temperatur des zum Eindampfen der Dispergierflüssigkeit verwendeten gasförmigen Fluids erweicht oder schmilzt.

14. Verfahren nach einem der vorhergehenden Ansprüche, dadurch gekennzeichnet, daß mindestens eine Kernteilchenmasse aus einem organischen Polymer, insbesondere ausgewählt aus einem Polyester, Polyethylen, Polystyrol, Polymethylmethacrylat, Cellulose, Nylon 6, Nylon 12, Teflon, einem Vinylchloridpolymer, einem Epoxyharz, einem Acrylharz, einem Methacrylharz, besteht oder dieses enthält, wobei die Tochterteilchen eine im wesentlichen gleichförmige Monoschicht bilden, die die Oberfläche jedes der Kernteilchen umhüllt, und wobei die Dispergierung in einer Dispergierflüssigkeit in einem Verhältnis von Tochterteilchen zu Kernteilchen stattfindet, welches zur Bildung der Monoschicht aus Tochterteilchen auf den Kernteilchen geeignet ist.

15. Verfahren nach Anspruch 14, dadurch gekennzeichnet, daß die Tochterteilchen aus einem mineralischen Pulver, insbesondere Talk, Kaolin, Glimmer, Vermiculit oder Kieselerde bestehen oder dieses enthalten.

16. Verfahren nach einem der vorhergehenden Ansprüche, dadurch gekennzeichnet, daß die Tochterteilchen ein Pigment, insbesondere Titanoxid, Zinkoxid, Eisenoxid, Eisentitanat, Ruß, Manganviolett, Chromoxid, Ultramarinblau oder Preußischblau, enthalten.

17. Verfahren nach einem der vorhergehenden Ansprüche, dadurch gekennzeichnet, daß die Tochterteilchen aus einem organischen Pulver, insbesondere einem Nylon- oder Polyethylenpulver bestehen oder dieses enthalten.

18. Verfahren nach einem der vorhergehenden Ansprüche, dadurch gekennzeichnet, daß die Kernteilchen und die Tochterteilchen ausgewählt sind aus den folgenden Paaren von Teilchenmassen: Nylon-Titanoxid, Nylon-Kieselerde, NylonEisenoxidgelb, Nylon-Ultramarinblau, Polystyrol-Titanoxid, Polyethylen-Titanoxid, Polyethylen-Eisenoxidgelb, PolyethylenEisenoxidschwarz, Polyethylen-Ultramarinblau.